# EUROPÄISCHE PATENTANMELDUNG

(11) **EP 1 293 504 A2**
(43) Veröffentlichungstag der Anmeldung: **19.03.2003**
(21) Anmeldenummer: 02025811.7
(22) Anmeldetag: 02.07.1997
(51) Int. Cl.: C07D 251/70, A61K 7/42

(54) **Triazinderivate als UV-Filter in kosmetischen Mitteln**

(30) Priorität: 08.07.1996 CH 170696
(62) Teilanmeldung aus: 97810425.5
(71) Anmelder: Ciba Specialty Chemicals Holding Inc., 4057 Basel (CH)
(72) Erfinder: Metzger, Georges, 68480 Moernach (FR); Reinehr, Dieter, 79400 Kandern (DE); Luther, Helmut, 79639 Grenzach-Wyhlen (DE)

(57) **Zusammenfassung**

Die vorliegende Erfindung betrifft die nichttherapeutische Verwendung von s-Triazinverbindungen der Formel 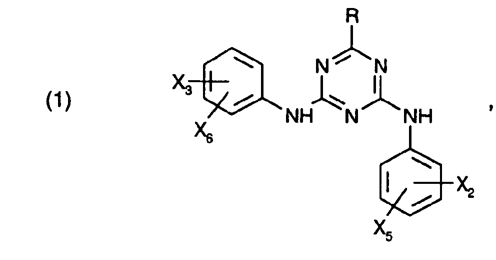 worin
- R: einen Rest der Formel (1a) 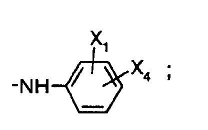
X₁, X₂ und X₃ -SO₂R₄;
- R₄: verzweigtes oder geradkettiges C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b); oder 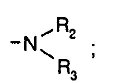
- R₂ und R₃: unabhängig voneinander Wasserstoff; geradkettiges oder verzweigtes C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b) 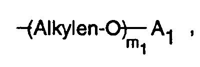 worin
- A₁: geradkettiges oder verzweigtes C₁-C₈-Alkyl; C₅-C₈-Cycloalkyl; gegebenenfalls mit einem oder mehreren C₁-C₄-Alkyl substituiertes C₆-C₁₂-Aryl; oder gegebenenfalls mit einem oder mehreren C₁-C₄-Alkyl substituiertes C₇-C₁₀-Aralkyl;
- m₁: 1 bis 10; und
- X₄, X₅ und X₆: Wasserstoff; oder Hxdroxy;
bedeuten, als UV-Filter in Sonnenschutzmitteln.

## Beschreibung

Die vorliegende Erfindung betrifft die Verwendung von Triazinderivaten als UV-Filter in kosmetischen Mitteln, insbesondere Sonnenschutzmitteln.

Die erfindungsgemäss verwendeten Triazinverbindungen entsprechen der Formel 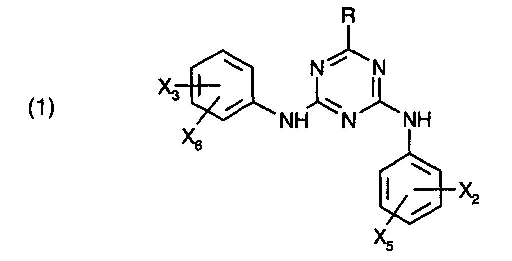 worin
- R: einen Rest der Formel (1a) 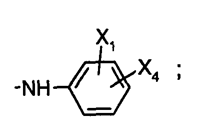
- X₁, X₂ und X₃: -SO₂R₄;
- R₄: verzweigtes oder geradkettiges C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b); oder 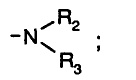
- R₂ und R₃: unabhängig voneinander Wasserstoff; geradkettiges oder verzweigtes C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b) 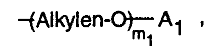 , worin
- A₁: geradkettiges oder verzweigtes C₁-C₈-Alkyl; C₅-C₈-Cycloalkyl; gegebenenfalls mit einem oder mehreren C₁-C₄-Alkyl substituiertes C₆-C₁₂-Aryl; oder gegebenenfalls mit einem oder mehreren C₁-C₄-Alkyl substituiertes C₇-C₁₀-Aralkyl;
- m₁: 1 bis 10; und
- X₄, X₅ und X₆: Wasserstoff; oder Hxdroxy;
bedeuten;

Geradkettiges und verzweigtes C₁-C₂₂-Alkyl sind z.B. Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl oder Eicosyl.

Beispiele für geradkettiges und verzweigtes C₁-C₁₈-Alkoxy sind z. B. Methoxy, Ethoxy, Propoxy, Isopropoxy, Butoxy, Isobutoxy, sek.-Butoxy, tert.-Butoxy, Pentoxy, Isopentoxy, n-Heptyloxy, n-Octyloxy, Isooctyloxy, n-Nonyloxy, Isononyloxy, Decyloxy, n-Dodecyloxy, Heptadecyloxy Octadecyloxy oder Eicosyloxy.

C₅-C₈-Cycloalkyl bedeutet z.B. Cyclopentyl, Cycloheptyl, Cyclooctyl und insbesondere Cyclohexyl.

Als Beispiele für C₆-C₁₂-Aryl sind insbesondere Phenyl, Naphthyl und Biphenylyl zu nennen.

Beispiele für C₇-C₁₀-Aralkyl sind Benzyl, Phenethyl, α-Methylphenethyl oder α,α-Dimethylbenzyl.

"Alkylen" in der Formel (1b) bedeutet eine bivalente Alkylengruppe mit 2 bis 5, vorzugsweise 2 bis 4 Kohlenstoffatomen. Es handelt sich dabei vorzugsweise um die -CH₂-CH₂-; -CH₂CH₂CH₂-; -CH₂-CH₂-CH₂-CH₂-; 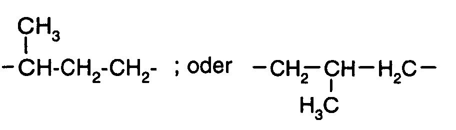 -Gruppe.

Unter diesen Alkylen-Gruppen sind ganz besonders die -CH₂-CH₂- und die 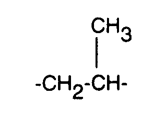 Gruppe bevorzugt.

Halogen bedeutet Fluor, Brom, lod oder vorzugsweise Chlor.

Vorzugsweise kommen Triazinverbindungen der Formel

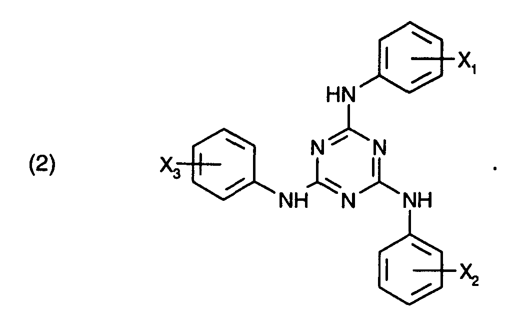

in Betracht. X₁, X₂ und X₃ haben dabei die in Formel (1) angegebene Be'deutung.

Im Vordergrund stehen Triazinverbindungen der Formel (1), worin X₁, X₂ und X₃ in ortho-Stellung zum Phenylaminorest des Triazins stehen, also Verbindungen der Formel 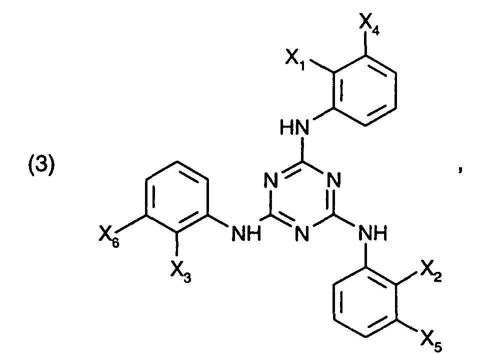 oder ganz besonders Triazinverbindungen der Formel (1), worin X₁, X₂ und X₃ in paraStellung zum Phenylaminorest des Triazins stehen, also Verbindungen der Formel 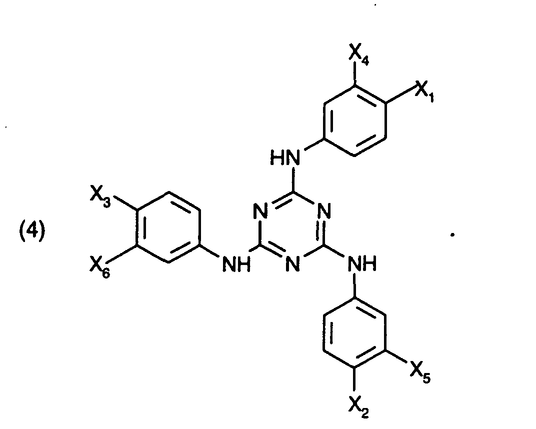 X₁, X₂, X₃, X₄, X₅ und X₆ haben dabei die in Formel (1) angegebene Bedeutung.

Insbesondere sind Triazinverbindungen der Formel (1) bevorzugt, worin
X₁, X₂ und X₃ die gleiche Bedeutung haben.

Im Vordergrund des Interesses stehen dabei Triazinverbindungen der Formel 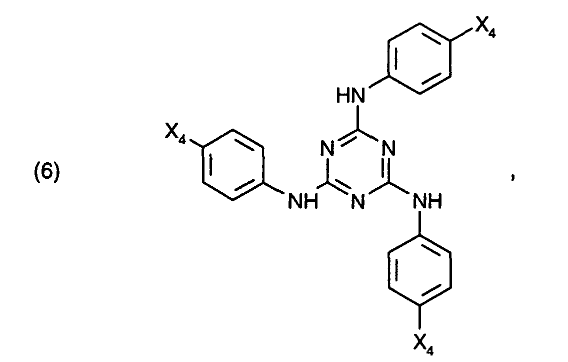 worin
- X₄: -SO₂R₁₀;
- R₁₀: geradkettiges oder verzweigtes C₁-C₂₂-Alkyl; oder -NH₂; bedeutet.

Die Herstellung der erfindungsgemässen Triazinverbindungen der Formel (1) erfolgt in an sich bekannter Weise, wie z.B. durch Umsetzung von 1 Mol Cyanurchlorid mit jeweils 1 Mol der entsprechenden Anilinverbindungen der Formeln

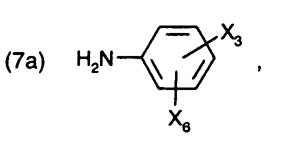

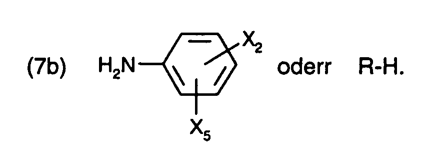

### R, X₂, X₃, X₅ und X₆ haben dabei die in Formel (1) angegebene Bedeutung

Die Reaktion wird gewöhnlich bei einer Temperatur von 50 bis 200°C in einem geeigneten Lösungsmittel durchgeführt.

Geeignete Lösungsmittel sind dabei zum Beispiel Acetonitril; Ketone, wie z.B. Aceton oder Methylethylketon; Ether, wie z.B. Diethylether, Diisopropylether, Tetrahydrofuran (THF), Dimethylformamid (DMF) oder Dioxan; aliphatische oder aromatische Kohlenwasserstoffe, wie z.B. Pentan, Heptan, Cyclohexan, Benzol, Toluol, Xylol oder Mischungen davon; oder aliphatische Carboxylsäureester wie z.B. Ethylacetat. Für das erfindungsgemässe Herstellungsverfahren wird als Lösungsmittel vorzugsweise Dimethylformamid (DMF) verwendet.

Die allgemeine Reaktion von Trihalogentriazinverbindungen, wie z.B. Cyanurfluorid oder Cyanurchlorid, bei der die drei Halogenatome durch Aminoreste ersetzt werden, sind bekannt und in der technischen Literatur, insbesondere der Fachliteratur, die sich mit Farbstoffen und optischen Aufhellern befasst, ausführlich beschrieben.

Die erfindungsgemässen Triazinverbindungen der Formel (1) eignen sich insbesondere als UV-A-Filter, d.h. zum Schützen von ultraviolett empfindlichen organischen Materialien, insbesondere der Haut und der Haare von Menschen und Tieren vor der schädigenden Einwirkung von UV-Strahlung. Diese Verbindungen eignen sich daher als Lichtschutzmittel in kosmetischen, pharmazeutischen und veterinärmedizinischen Präparaten. Die Verbindungen können sowohl gelöst als auch im mikronisierten Zustand verwendet werden.

Einen weiteren Erfindungsgegenstand bildet daher ein kosmetisches Präparat, enthaltend mindestens eine Verbindung der Formel (1), sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die erfindungsgemässen Triazinverbindungen, die gewöhnlich wasserunlöslich sind, können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew. %, bezogen auf die eingesetzte Triazinverbindung, einer Mahlhilfe, wie z.B. eines alkylierten Vinylpyrrolidon-Polymers, eines Vinylpyrrolidon-Vinylacetat-Copolymers, eines Acylglutamates oder insbesondere eines Phospholipids durchgeführt. Das so erhaltene Nanopigment wird in eine übliche Sonnenschutz-Rezeptur eingearbeitet. Die Herstellung von O/W- oder W/O-Emulsionen mit einem oder mehreren Pigmenten, auch in Gegenwart eines oder mehrerer öl- oder wasserlöslicher UV-Absorber, erfolgt nach bekannten Verfahren für die Herstellungen von Sonnenschutz-Emulsionen.

Die kosmetischen Zubereitungen können neben den erfindungsgemässen Triazinverbindungen auch noch einen oder mehrere weitere UV-Schutzstoffe, wie z.B. Benzophenone, p-Methoxyzimtsäureester, Dibenzoylmethanderivate, Benzylidencampherderivate, p-Aminobenzoesäureester, Salicylsäurederivate, Diphenylacrylat-Derivate, Terephthalyden-Dicamphersulfonsäure, Octyltriazone, Phenylbenzimidazol-Sulfonsäure, Menthylanthranilat, TiO₂ (unterschiedlich umhüllt), ZnO, Mica, Benzotriazole oder Vinylgruppen enthaltende Amide oder Zimtsäureamide enthalten. Solche Schutzstoffe sind z.B. in der GB-A-2,286,774 beschrieben oder auch aus Cosmetics & Toiletries (107), 50ff (1992) bekannt.

Die erfindungsgemässe kosmetische Zubereitung enthält z.B. 0,1 bis 15, vorzugsweise 0,5 bis 10 Gew.-%, bezogen auf das Gesamtgewicht der Zusammensetzung, einer erfindungsgemässen Triazinverbindung der Formel (1) oder eines Gemisches aus diesen Triazinverbindungen und einen kosmetisch verträglichen Hilfsstoff.

Die Herstellung der kosmetischen Zusammensetzung kann durch physikalisches Mischen des oder der Triazinverbindungen mit dem Hilfsstoff durch gewöhnliche Methoden, wie z.B. durch einfaches Zusammenrühren der Einzelkomponenten erfolgen.

Die erfindungsgemässe kosmetische Zubereitung kann als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikonöl, einen Fettsäureester oder einen Fettalkohol. Bevorzugte Mono- oder Polyole sind Ethanol, Isopropanol, Propylenglykol, Hexylenglycol, Glycerin und Sorbitol.

Für die erfindungsgemässen kosmetischen Formulierungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-ÖI; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die kosmetische Formulierung kann auch weitere Komponenten wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe enthalten.

Die erfindungsgemässen kosmetischen Formulierungen zeichnen sich durch eine hohe UV-Absorption aus und bieten daher einen sehr guten Schutz der menschlichen Haut gegen den schädigenden Einfluss von Sonnenlicht.

## Patentansprüche

1. Nichttherapeutische Verwendung der Triazinverbindungen der Formel 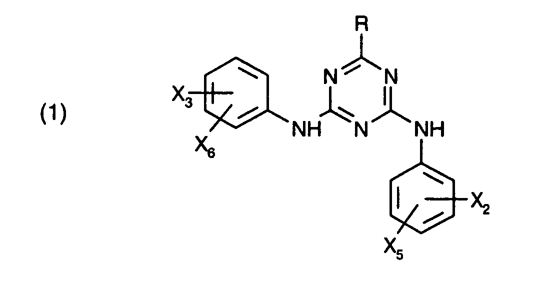 worin
R einen Rest der Formel (1a) 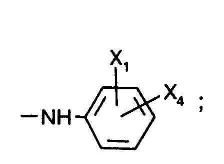
X₁, X₂ und X₃ -SO₂R₄;
R₄ verzweigtes oder geradkettiges C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b); oder 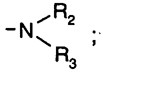
R₂ und R₃ unabhängig voneinander Wasserstoff; geradkettiges oder verzweigtes C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b) 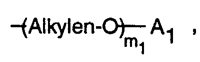 ,worin
A₁ geradkettiges oder verzweigtes C₁-C₈-Alkyl; C₅-C₈-Cycloalkyl; gegebenenfalls mit einem oder mehreren C₁-C₄-Alkyl substituiertes C₆-C₁₂-Aryl; oder gegebenenfalls mit einem oder mehreren C₁-C₄-Alkyl substituiertes C₇-C₁₀-Aralkyl; und
m₁ 1 bis 10; und
X₄, X₅ und X₆ Wasserstoff; oder Hxdroxy; bedeuten;
zum Schützen von menschlichen und tierischen Haaren und der Haut vor der schädigenden Einwirkung von UV-Strahlung.

2. Verwendung nach Anspruch 1, **dadurch gekennzeichnet, dass** "Alkylen" in Formel (1b) eine Alkylengruppe darstellt, die 2 bis 4 Kohlenstoffatome aufweist.

3. Verwendung nach Anspruch 1 oder 2, **dadurch gekennzeichnet, dass** die Alkylengruppe bivalente Reste der Formeln -CH₂-CH₂-; -CH₂CH₂CH₂-; -CH₂-CH₂-CH₂-CH₂-; 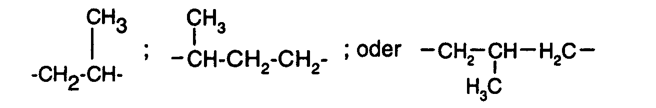 darstellt.

4. Verwendung nach einem der Ansprüche 1 bis 3, **dadurch gekennzeichnet, dass** sie der Formel 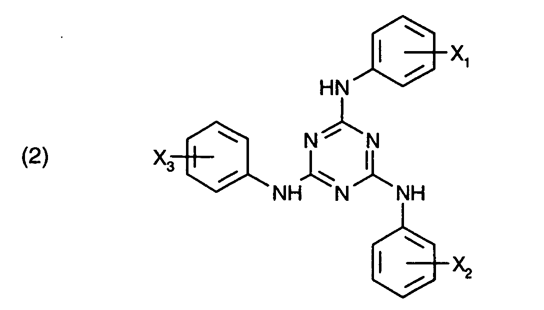 entsprechen, worin
X₁, X₂ und X₃ die in Anspruch 1 angegebene Bedeutung haben.

5. Verwendung nach einem der Ansprüche 1 bis 3, **dadurch gekennzeichnet, dass** sie den Formeln 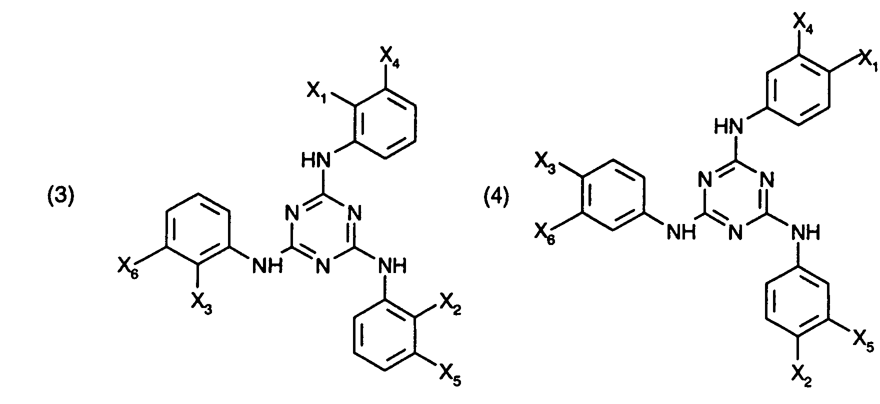 oder 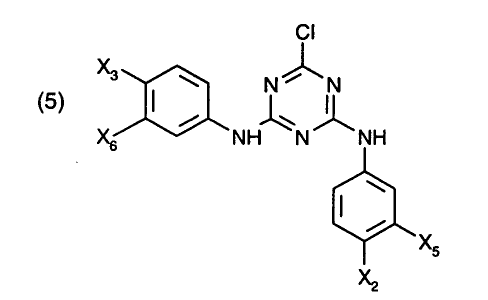 entsprechen,
wobei
X₁, X₂, X₃, X₄, X₅ und X₆ die in Anspruch 1 angegebene Bedeutung haben.

6. Verwendung nach einem der Ansprüche 1 bis 5, **dadurch gekennzeichnet, dass**
X₁, X₂ und X₃ die gleiche Bedeutung haben.

7. Verwendung nach Anspruch 6, **dadurch gekennzeichnet, dass** die Triazinverbindungen der Formel 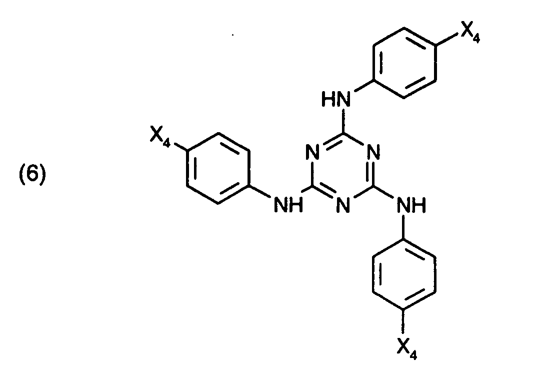 entsprechen, worin
X₄ -SO₂R₁₀; und
R₁₀ geradkettiges oder verzweigtes C₁-C₂₂-Alkyl; oder -NH₂; bedeuten.

8. Kosmetisches Präparat, enthaltend mindestens eine oder mehrere Verbindungen nach den Ansprüchen 1 bis 7 sowie kosmetisch verträgliche Träger- oder Hilfsstoffe.

9. Präparat nach Anspruch 8, **dadurch gekennzeichnet, dass** es weitere UV-Schutzstoffe enthält.

10. Präparat nach Anspruch 8 oder 9, **dadurch gekennzeichnet, dass** es als weitere UV-Schutzstoffe Benzophenone, p-Methoxyzimtsäureester, Dibenzoylmethanderivate, Benzylidencampherderivate, p-Aminobenzoesäureester, Salicylsäuraederivate, Diphenylacrylat-Derivate, Terephthalyden-Dicamphersulfonsäure, Octyltriazone, Phenylbenzimidazol-Sulfonsäure, Menthylanthranilat, TiO₂ (unterschiedldich umhüllt), ZnO, Mica, Benzotriazole oder Vinylgruppen enthaltende Amide oder Zimtsäureamide enthält.
